# EUROPEAN PATENT SPECIFICATION

(11) **EP 4 225 229 B1**
(45) Date of publication and mention of the grant of the patent: **26.08.2026**
(21) Application number: 21806882.3
(22) Date of filing: 05.10.2021
(51) Int. Cl.: A61F 5/44, A61F 5/442, A61F 5/453, A61F 5/455

(54) **FLUID TRANSFER ASSEMBLIES**
FLÜSSIGKEITSÜBERTRAGUNGSVORRICHTUNGEN
ENSEMBLES DE TRANSFERT DE FLUIDES

(30) Priority: 07.10.2020 US 202063088506 P
(43) Date of publication of application: 16.08.2023
(73) Proprietor: Purewick Corporation, El Cajon, California 92020 (US)
(72) Inventor: MINCHEW, Ana, Covington, Georgia 30014 (US); GLOECKNER, Claire, Lilburn, Georgia 30047 (US); HUGHETT, SR., James David, Conyers, Georgia (US); SIDDIQUI, Shahab, Lawrenceville, Georgia 30044 (US); KULKARNI, Vinayaka, Bangalore, Karnataka 56007 (IN)
(74) Representative: Hoffmann Eitle
(86) International application number: PCT/US2021/053593
(87) International publication number: WO 2022/076427

(56) References cited:
- EP-A1- 2 676 643
- EP-A2- 2 997 950
- US-A1- 2012 233 761

## Description

### BACKGROUND

An individual may have limited or impaired mobility such that typical urination processes are challenging or impossible. For example, the individual can have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing. In such cases, fluid collection devices are used to collect fluid from the individual.

Currently, the disposal of collected fluid and the cleaning of fluid collection devices is done manually, potentially exposing the user or caregiver to the fluids collected from the individual. The user or caregiver can be required to perform extensive cleaning procedures. The cleaning procedures can cause further exposure to the contents of the container. US 2012/233761 A1 discloses an automatic wheelchair-type feces and urine cleaner.

### SUMMARY

According to a first aspect, there is provided a fluid transfer assembly according to claim 1.

Features from any of the disclosed embodiments can be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a block diagram of a fluid transfer assembly, according to an embodiment.
**FIG. 2** is an isometric view of a fluid transfer assembly, according to an embodiment.
**FIG. 3** is an isometric view of the fluid transfer assembly of FIG. 2, according to an embodiment.
**FIG. 4** is an isometric view of a refilling process of a cleaning device, according to an embodiment.
**FIG. 5** is a cross-sectional diagram of cleaning device, according to an embodiment.
**FIG. 6** is a block diagram of a fluid transfer assembly, according to an embodiment.
**FIG. 7** is a process flow diagram for a fluid transfer assembly, according to an embodiment.

### DETAILED DESCRIPTION

Embodiments disclosed herein are related to devices, systems, and methods of using fluid transfer devices and systems. Specifically, the devices, systems, and methods described herein relate to a fluid transfer assemblies for the cleaning of fluid collection devices. Male and female urine collection devices frequently require emptying and cleaning. The disposal of urine and the cleaning of collection devices has traditionally been performed manually by a user or caregiver, which unnecessarily exposes the individual to the contaminants.

A technical effect is achieved by a cleaning device that is in fluid communication with a part of a urine collection device, and that includes a chamber configured to store a liquid for cleaning; and a pump. The technical advantage is that it allows for automatically cleaning urine collection devices, and therewith exposure of humans to contaminants can be reduced or even eliminated. This is an improvement over traditional methods of manually cleaning the urine collection devices because it reduces or eliminates exposure to contaminants.

According to one or more embodiments, a urine transfer assembly can include a fluid collection device and a cleaning device. The fluid collection device can include a container defined by a fluid impermeable barrier for receiving and storing a fluid. The fluid collection device can be configured to collect fluid(s) from an individual. The fluid collected by the fluid collection device can include urine. The fluid(s) collected by the fluid collection device can also include at least one of vaginal discharge, penile discharge, reproductive fluids, blood, sweat, or other bodily fluids.

To reduce maintenance of the collection device and exposure to the fluids in the collection device, the fluid transfer assembly can include a cleaning device that is capable of forming a fluid connection with the container of the fluid connection device. The cleaning device can include a chamber of air and/or liquid and a pump configured to modulate the pressure within the container of the collection device sufficient to expel the content thereof and clean the container.

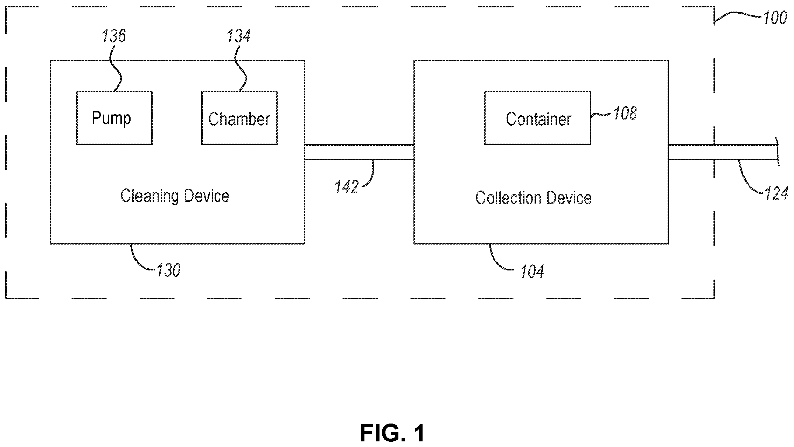

**FIG. 1** is a block diagram of a fluid transfer assembly 100, according to an embodiment. The fluid transfer assembly 100 can include a collection device 104 and a cleaning device 130. The collection device 104 can be a fluid collection device and can include a container 108 for collecting fluid, such as urine, from an individual.

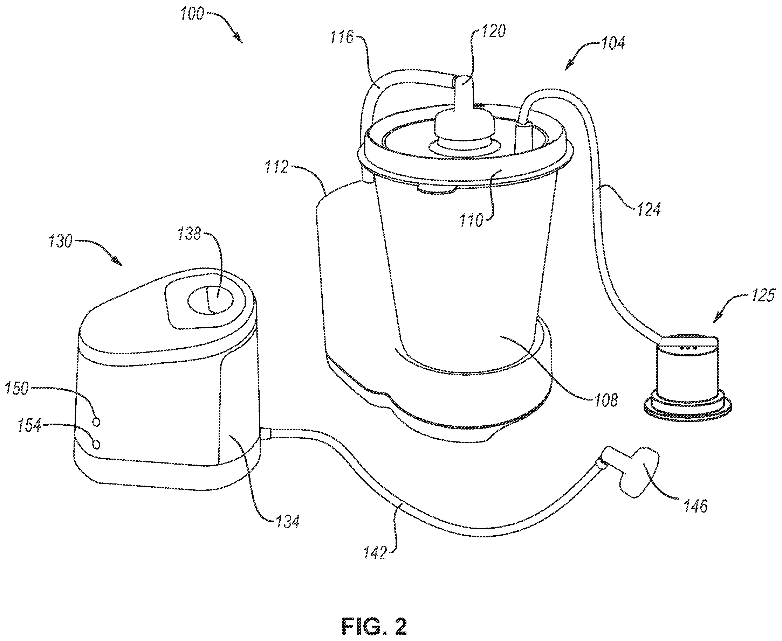

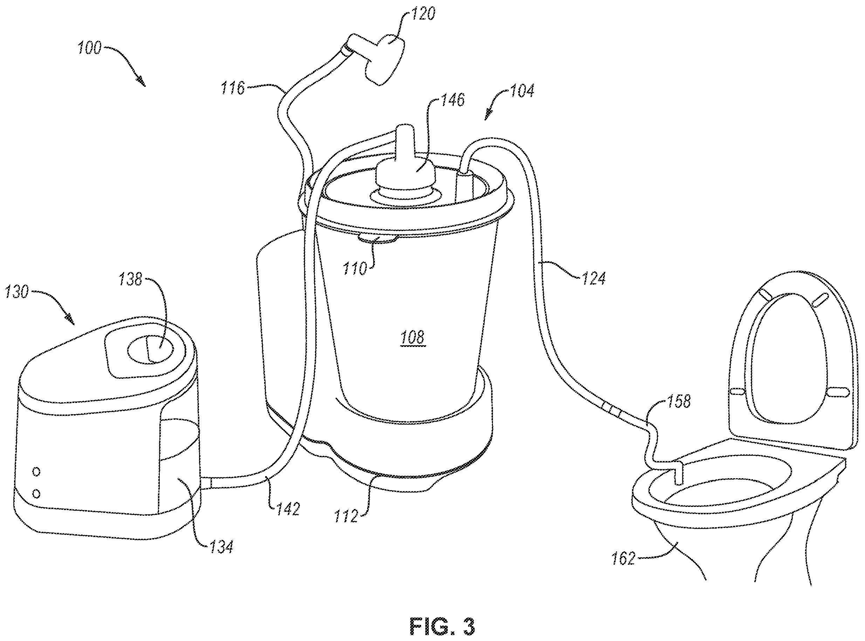

The fluid transfer assembly 100 can include an outlet conduit 124 that is in fluid communication with the container 108 of the collection device 104. The cleaning device 130 can include a chamber 134 for storing a gas or liquid, and a pump 136 for modulating the pressure within the chamber 134 and/or the container 108. The cleaning device 130 and the collection device 104 can be fluidly connected via a conduit 142. In some embodiments, the cleaning device 130 and the collection device 104 are contained within a common housing of the fluid transfer assembly 100. In some embodiments, the cleaning device 130 and the collection device 104 define separate and distinct housings. Further details of the fluid transfer assembly 100 are provided below with reference to FIGS. 2 and 3.

**FIG. 2** is an isometric view of the fluid transfer assembly 100, according to an embodiment. The container 108 can be removably attached to a base 112 of the collection device 104. The container 108 can define an opening near a top of the container 108. A lid 110 can be configured to close and/or seal the opening at the top of the container 108. The lid 110 can define one or more apertures or ports to fluidly connect the internal volume of the container 108 with external devices or environments. It will be understood that in some embodiments, the lid 110 is excluded and the container 108 can define a substantially continuous volume with ports or apertures to connect the internal volume of the container 108 with the outside environment.

The base 112 can include a housing configured to store or house internal electrical and mechanical components. For example, the base 112 can house a pump (not shown in FIG. 2) configured to modulate pressure within the container 108. In some embodiments, the pump is configured to pull a vacuum in the container 108, for example, to draw fluid into the container 108 via conduit 124. The urine can be collected using a urine collection apparatus 125 that is positioned at least proximate to a uretheral opening. Examples of fluid collection apparatuses that are configured to collected bodily fluids from a male or female urethral opening and methods of using such fluid collection assemblies are disclosed in International Application No. PCT/US2020/42262 filed on July 14, 2020, U.S. Patent Publication No. 2019/0282391 filed on June 6, 2019, and U.S. Provisional Patent Application No. 63/047,374 filed on July 2, 2020. Other embodiments of fluid impermeable barriers, fluid permeable membranes, fluid permeable supports, chambers, and their shapes and configurations are disclosed in U.S. Patent Application No. 15/612,325 filed on June 2, 2017; U.S. Patent Application No. 15/260,103 filed on September 8, 2016; and U.S. Patent No. 10,225,376 filed on June 1, 2017.

For example, the pump can be operable to draw urine, expelled into the urine collection apparatus 125 by a subject, into the container 108 through the conduit 124. In some embodiments, the pump is a reversible pump, configured to selectively create a vacuum in the container 108 or generate positive pressure within the container 108. For example, the pump can push air into the container 108 to expel the fluid out of the container 108 via conduit 124. The pump can be in fluid communication with the internal volume of the container 108 via a conduit 116. The conduit 116 can be coupled to the lid 110 of the container 108 by a coupling element 120. The coupling element 120 can be configured to removably attach the conduit 116 to the lid 110. The coupling element 120 can be configured to create an air/liquid tight seal between the conduit 116 and the lid 110.

The cleaning device 130 can be a stand-alone unit that is independent from the collection device 104. The cleaning device 130 can define a chamber 134 suitable for containing liquid and/or air. The cleaning device 130 can include a lid 138 to access the internal volume of the chamber 134. The chamber 134 can be in fluid communication with the container 108 of the collection device 104 via a conduit 142. A first end of the conduit 142 can form a sealed attachment with the chamber 134, and a second end of the conduit 142 can include a coupling element 146 configured to form an air/liquid tight seal with the lid 110 (as shown in FIG. 3). The coupling element 146 of the cleaning device 130 can be substantially similar to the coupling element 120 of the collection device 104.

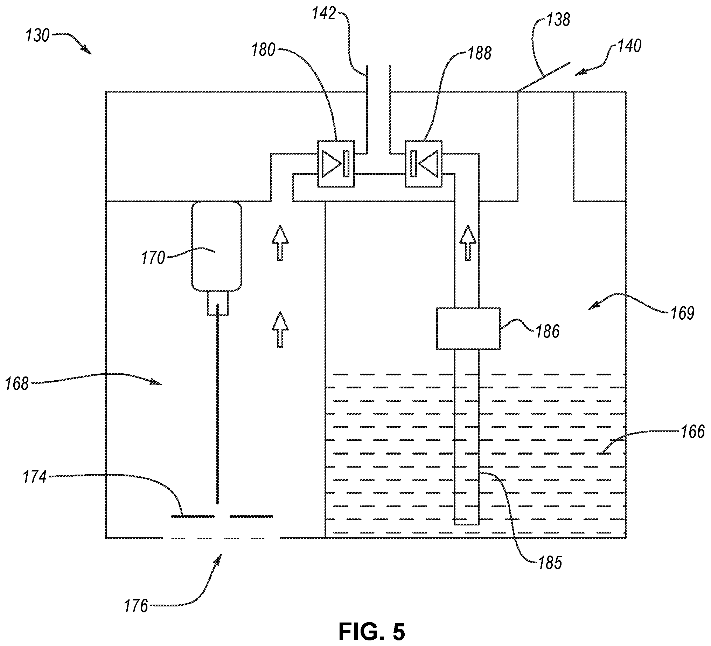

As described in greater detail with reference to FIG. 5, the cleaning device 130 can include a one or more pumps or motors (not shown in FIG. 2) to modulate the pressure within the chamber 134, and consequently within the container 108 of the collection device 104. The cleaning device 130 can include one or more buttons for the operation of the cleaning device. According to one embodiment, the cleaning device 130 can include a first button 150 that activates the cleaning device 130 to provide high pressure air to the container 108 sufficient to expel the contents of the container through the conduit 124. A second button 154 can activate the cleaning device 130 to provide a cleaning liquid, such as water or a cleaning solution, to flush out and clean the container 108. Further details of the modes of operation of the cleaning device are provided below with reference to FIGS. 3-7.

**FIG. 3** is an isometric view of the fluid transfer assembly 100 with the cleaning device 130 attached to the collection device 104. The embodiment illustrated in FIG. 3 illustrates a coupling of the cleaning device 130 and the collection device 104. When it is desired to empty the contents of the container 108 and/or clean the container 108, the coupling element 120 of the base 112 can be removed from the lid 110 and replaced with the coupling element 146 of the cleaning device 130, thereby bringing the chamber 134 and the container 108 into fluid communication via conduit 142. A distal end 158 of the conduit 124 can be positioned to empty contents of the container 108 into a waste receptacle, such as a toilet 162.

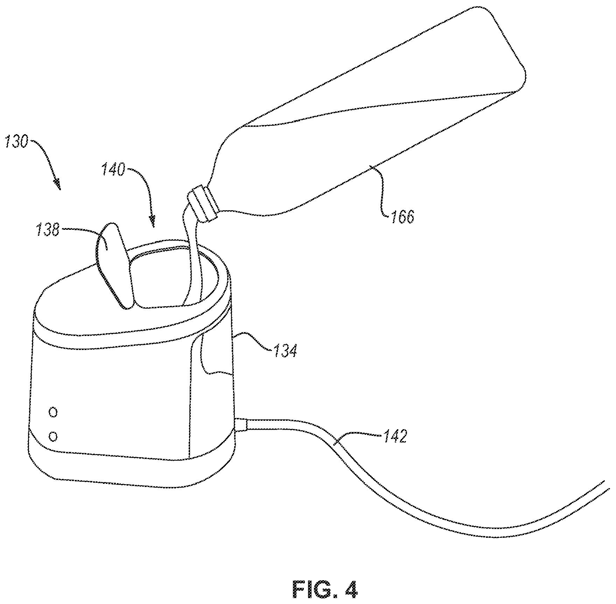

As discussed in greater detail herein, according to some embodiments, once attached to the collection device 104, the cleaning device 130 produces a positive pressure in the chamber 134 which pushes air into the container 108. The positive pressure generated in the container 108 then forces the contents of the container 108, such as urine, through the conduit 124 and into the toilet 162 or other waste disposal unit. The cleaning device 130 can then pump a cleaning liquid, such as water or a cleaning solution, into the container 108. Once the cleaning liquid is transferred from the chamber 134 to the container 108, the cleaning device 130 can again provide a positive air pressure into the container 108 to force the cleaning liquid from the container 108 and into the toilet 162, via conduit 124. Further details of the fluid transfer assembly are provided below with reference to FIGS. 4 and 5.

**FIG. 4** is an isometric view of a refilling process of the cleaning device 130. The internal volume of the chamber 134 can be made accessible by opening the lid 138 to reveal an aperture 140. A cleaning liquid 166 can be poured into the chamber 134 through the aperture 140. In some embodiments, the cleaning liquid 166 is added to the chamber 134 after the initial step of providing air pressure to expel urine from the collection device 104. The cleaning liquid 166 can include a cleaning solvent, such as at least one of isopropyl alcohol, glycerin, propylene glycol, or bleach. In some embodiments, the cleaning liquid 166 is stored is a separate compartment of the chamber 134, as discussed below in FIG. 5.

**FIG. 5** is a cross-sectional diagram of the cleaning device 130, according to an embodiment. The cleaning device 130 can define two or more independent and isolated compartments. A first compartment 168 can define an internal volume of air. The first compartment 168 can house a motor 170 configured to transfer air to the conduit 142. The internal volume of the first compartment 168 can be in fluid communication with the outside atmosphere via a vent 176 formed in a wall of the first compartment 168. The motor 170 can be configured to drive a fan or impeller 174 to draw in atmospheric air thought the vent 176 and generate high pressure air to push into the conduit 142. A first valve 180, such as a one-way valve or check valve, can be positioned between the internal volume of the first compartment 168 and the conduit 142. The first valve 180 can be configured to allow air flow toward the conduit 142 while preventing air flow from the conduit 142 toward the first compartment 168.

The second compartment 169 of the cleaning device 130 can define an internal volume configured to store liquid, such as cleaning liquid 166. The second compartment 169 can house a pump 186 configured to transfer the cleaning liquid 166 from the second compartment 169 to the conduit 142. The pump 186 of the second compartment 169 can include any of a variety of pumps, such as a diaphragm pump. The pump 186 can include multiple flow rates that allow a user or wearer to select a desired or preferred flow rate. For example, a higher flow rate can be used to obtain a more thorough flushing of the container 108 with the cleaning liquid 166.

A tube 185 can be in fluid communication with the pump 186 and can extend into the cleaning liquid 166, such that when activated the pump 186 draws the cleaning liquid 166 into the tube 185. A second valve 188, such as a one-way valve or check valve, can be positioned between the internal volume of the second compartment 169 and the conduit 142. The second valve 188 can be configured to allow the cleaning liquid 166 to flow toward the conduit 142 while preventing flow from the conduit 142 toward the second compartment 169. Further, the second valve 188 can be configured to prevent air from the first compartment 168 from entering the second compartment 169. Likewise, the first valve 180 can prevent the cleaning liquid 166 from entering the first compartment 168.

According to an embodiment, in a first mode, the cleaning device 130 is configured to expel fluid in the container 108 of the fluid collection device 104 by pushing high pressure air into the container 108. In the first mode, the motor 170 can be activated to actuate the fan 174 to push air passed the first valve 180 and to the conduit 142. The high pressure air travels through the conduit 142 to the container 108. The positive pressure generated in the container can then push out the fluid in the container 108 via conduit 124.

A second mode of the cleaning device 130 can be configured to flush the container 108 with the cleaning liquid 166. According to an embodiment, upon activation of the pump 186, a vacuum formed in the tube 185 draws the cleaning liquid 166 from the second compartment 169 and pushes the cleaning liquid 166 into the container 108 via the conduit 142.

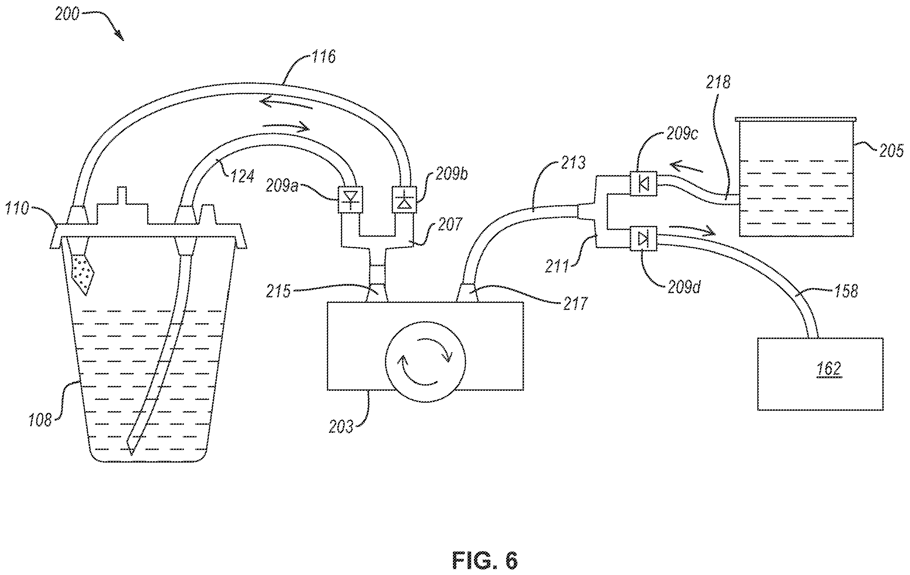

Once a desired amount of cleaning liquid 166 has been transferred from the second compartment 169 to the container 108, the cleaning device 130 can again enter the first mode in order to generate positive pressure within the container 108 to expel the cleaning liquid 166 through conduit 124. In some embodiments, selection of the modes is done by a user, for example by actuating buttons 150 and 154 as shown in FIG. 2. In some embodiments, the cleaning device 130 is configured to automatically switch modes based on, for example, a time duration or a detected pressure within the chamber 134. Further details of example fluid transfer assemblies are provided below with reference to FIG. 6.

**FIG. 6** shows a block diagram of a fluid transfer assembly 200, according to an embodiment. The assembly 200 can, in terms of components and operation, be substantially similar to the apparatus 100 discussed above. The assembly 200 can include a container 108, a pump 203, and a reservoir 205. The container 108 can be configured to collect and store fluid from an individual. For example, conduit 124 can be in fluid communication with the individual. When it is desired to empty and clean the container 108, the conduit 124 can be removed from the individual and attached to the pump 203.

The pump 203 can include any of a variety of pumps, such as a diaphragm pump. The pump 203 can include multiple flow rates that allow a user to select a desired or preferred flow rate. The pump 203 can represent a removable or modular pump that allows a user to remove the pump 203 and attach a different pump to the system 200. The pump 203 also can include a portable pump that is transportable by a user.

As discussed in greater detail below, the pump 203 can be configured to pull a vacuum in the container 108 to pull fluid, such as urine from the container 108. In some embodiments, the pump 203 can be configured to provide a positive pressure within the container 108. The pump 203 can be a reversible pump, configured to selectively pull fluid or air in and push fluid or air out of the container 108. For example, in a first mode, the pump 203 can be configured to draw a vacuum in the container, and in a second mode, the pump 203 can be configured to provide positive pressure in the container 108.

In some embodiments, the pump 203 can include a first port 215 and a second port 217. The assembly 200 can include a first tee joint 207 coupled to the first port 215 and a second tee joint 211 coupled to the second port 217. The internal volume of the container 108 can be in fluid communication with the first tee joint 207 via conduits 116 and 124. A first check valve 209a can be positioned between the conduit 124 and the first tee joint 207. The first check valve 209a can be configured to allow fluid or air to flow from the container 108 into the pump 203 and prevent fluid or air from flowing from the pump 203 into the container 108 via conduit 124. The internal volume of the container 108 can further be in fluid communication with the first tee joint 207 via a conduit 116. A second check valve 209b can be positioned between the conduit 116 and the first tee joint 207. The second check valve 209b can be configured to allow fluid or air to flow from the pump 203 into the container 108 and prevent fluid or air from flowing from the container 108 into the pump 203 via conduit 116.

The second port 217 of the pump 203 can be connected to the second tee joint 211 via a conduit 213. The reservoir 205 can be in fluid communication with the second tee joint 211 via conduit 218. A third check valve 209c can be positioned between the second tee joint 211 and the reservoir 205. The third check valve 209c can be configured to allow fluid or air to flow from the reservoir 205 into the pump 203, and prevent fluid or air from flowing from the pump 203 into the reservoir 205 via the conduit 218. A waste receptacle 162, such as a toilet, can be in fluid communication with the second tee joint 211 via a conduit 158. A fourth check valve 209d can be placed between the conduit 158 and the second tee joint 211. The fourth check valve 209d can be configured to allow fluid or air to flow from the pump 203 into the waste receptacle 162, and prevent fluid or air from flowing into the pump 203 via conduit 158.

In the first mode, upon activation of the pump 203, the first port 215 can draw air or fluid into the pump 203 and the pump 203 can force out the air or fluid out of the second port 217. During the first mode, the pump 203 pulls a vacuum in the container 108 to draw fluids from the container 108 through the conduit 124, passed the first check valve 209a and into the pump 203. The drawn fluid is then pushed by the pump 203 through the conduit 213 to the second tee joint 211 and passed the fourth check valve 209d where it is expelled from the conduit 158 into the toilet 162.

During the second mode, the pump 203 can be reversed from the first mode. In the second mode, the pump 203 pulls a vacuum in the reservoir 205 to draw a cleaning liquid into the pump 203 through the second port 217. The cleaning liquid can then be pushed out of the first port 215 of the pump 203 into the conduit 116 and into the container 108 to clean and sanitize the internal volume of the container 108. Thereafter, the pump 203 can be switched back to the first mode in order to draw the cleaning liquid from the container 108 and expel the cleaning solution into the toilet 162.

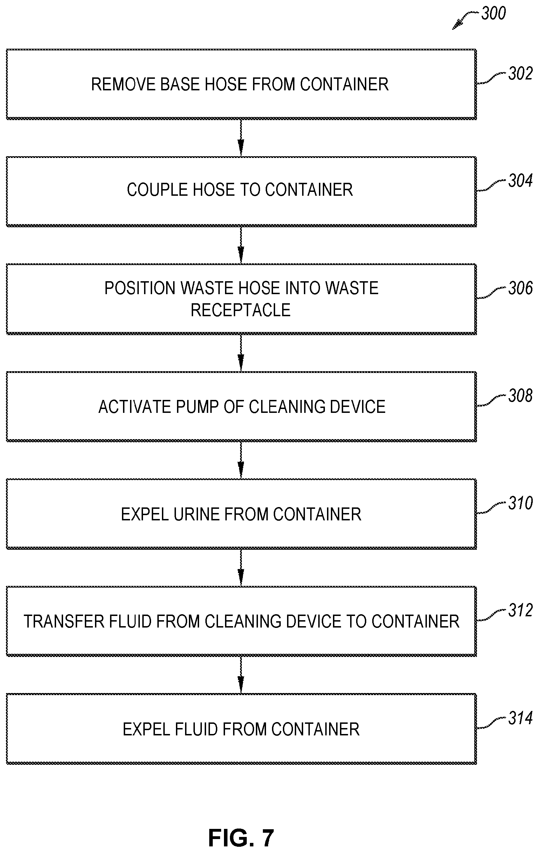

**FIG. 7** is a process flow diagram of a process 300 for a fluid transfer assembly, according to an embodiment. Some or all of the acts of process 300 can be performed using fluid transfer assemblies 100 or 200 discussed above. The process 300 can be initiated in response to a desire or need to empty and clean a container of a urine collection device. At act 302, a hose, tube, or conduit that is coupled to a base pump of a urine collection device can be detached from the container. At act 304, a hose, coupled at one end to the cleaning device, can be coupled at its second end to the container, for example at the same location from which the base hose was removed in act 302. At act 306, a waste hose can be positioned to expel urine into a waste receptacle, such as a toilet. At act 308, a pump can be activated to modulate pressure within the container of the collection device. According to an embodiment, upon activation of the pump a positive pressure is generated in the chamber of the cleaning device and/or the container of the urine collection device, as discussed with reference to the fluid transfer assembly 100. In some embodiments, upon activation of the pump, a vacuum is pulled in the container, as discussed with reference to the fluid transfer assembly 200.

At act 310, the modulated pressure created by the pump at step 308 causes the urine within the container to be expelled from the container into a waste receptacle (*e.g.,* a toilet) via the waste hose. In some embodiments, upon activation, the pump transfers fluid into the container which ultimately flushes the urine out of the container, as opposed to being pushed out by high pressure air. At act 312, fluid, such as water or cleaning solution, is transferred from the cleaning device to the container. This can be in response to the cleaning device or pump switching modes. At act 314, the cleaning fluid is expelled from the container by positive pressure or by a vacuum generated by the pump of the cleaning device. This can be in response to the cleaning device or pump again changing modes. At this stage, the container has been emptied and cleaned and the assembly can be returned to its original assembly. For example, the hose of the cleaning device can be detached from the container and the hose of the base unit can be reattached.

Acts 302, 304, 306, 308, 310, 312, and 314 of the process 300 are for illustrative purposes. For example, the acts 302, 304, 306, 308, 310, 312, and 314 of the process 300 can be performed in different orders, split into multiple acts, modified, supplemented, or combined. In an embodiment, one or more of the acts 302, 304, 306, 308, 310, 312, and 314 can be omitted from the process 300.

While the specific embodiments discussed herein have been focused on the fluid transfer of a fluid collection device, it will be understood that the methods, systems, and apparatuses discussed herein could be applied to other devices. For example, a continuous positive airway pressure (CPAP) mask can be contained within a sealed vessel and sterilized using the cleaning device as described herein.

While various aspects and embodiments have been disclosed herein, other aspects and embodiments are contemplated. The various aspects and embodiment disclosed herein are for purposes of illustration and are not intended to be limiting.

## Claims

1. A fluid transfer assembly (100;200) for the collection and disposal of urine, the fluid transfer assembly comprising:
a urine collection device (104) including:
a container (108) configured to store urine collected from a urine collection apparatus (125) positioned proximate to a urethral opening of an individual, the container defining a port coupleable to the urine collection apparatus; and
an outlet configured to expel the urine from the container; **characterised by**
a cleaning device (130) in fluid communication with the container (108), the cleaning device (130) including:
a chamber (134;169;205) configured to store a cleaning liquid (166); and
a pump (136;186;170;203) configured to modulate pressure within the container (108) to provide a positive gas pressure in the container to expel the urine through the outlet (124).

2. The fluid transfer assembly of claim 1, further comprising an outlet conduit (124) coupled to the outlet of the urine collection device and configured to be in fluid communication with a waste receptacle (162).

3. The fluid transfer assembly of any of the preceding claims, wherein the cleaning liquid includes at least one of water or a disinfectant.

4. The fluid transfer assembly of any of the preceding claims, wherein the cleaning device includes a first motor (170) configured to provide positive air pressure to the container of the urine collection device, and a second motor (186) configured to transfer the liquid from the chamber to the container.

5. The fluid transfer assembly of any of the preceding claims, wherein the cleaning device includes a battery configured to power the cleaning device.

6. The fluid transfer assembly of any of the preceding claims, wherein the cleaning device is configured to receive power via the urine collection device.

7. The fluid transfer assembly of any of the preceding claims, wherein the urine collection device includes a second pump configured to be in fluid communication with the cleaning device.

8. The fluid transfer assembly of any of the preceding claims, wherein the urine collection device includes a port coupleable to a vacuum tube and also to a conduit of the cleaning device to receive the cleaning liquid from the cleaning device.

9. The fluid transfer assembly of any of the preceding claims, wherein the pump is configured to provide positive pressure to the container to expel the cleaning liquid from the container effective to empty the container of the cleaning liquid.

10. The fluid transfer assembly of any of the preceding claims, wherein the urine collection device and the cleaning device are positioned in a common housing.

11. The fluid transfer assembly of claim 1, wherein the cleaning device further comprises an air chamber including a volume of air.

12. The fluid transfer assembly of claim 11, wherein the air chamber is in fluid communication with the atmosphere.

13. The fluid transfer assembly of any of claims 11 or 12, further comprising at least one valve (180;188) configured to regulate flow of the liquid or air through an outlet of the cleaning device (130).

14. The fluid transfer assembly of any of claims 11 to 13, wherein the chamber, the air chamber, and the pump are disposed within a housing.

15. The fluid transfer assembly of any of claims 11-14, wherein the pump is positioned in a tank of the cleaning device, the tank configured to store liquid.

## Patentansprüche

1. Flüssigkeitsübertragungsanordnung (100; 200) zur Sammlung und Entsorgung von Urin, wobei die Flüssigkeitsübertragungsanordnung umfasst:
eine Urinsammelvorrichtung (104), einschließend:
einen Behälter (108), der dazu ausgebildet ist, Urin zu speichern, der von einer Urinsammeleinrichtung (125) gesammelt wird, die in der Nähe einer Harnröhrenöffnung einer Person positioniert ist, wobei der Behälter einen Anschluss definiert, der mit der Urinsammeleinrichtung koppelbar ist; und
einen Auslass, der dazu ausgebildet ist, den Urin aus dem Behälter zu entlassen;
**gekennzeichnet durch**
eine Reinigungsvorrichtung (130), die mit dem Behälter (108) in Fluidverbindung steht, wobei die Reinigungsvorrichtung (130) einschließt:
eine Kammer (134; 169; 205), die dazu ausgebildet ist, eine Reinigungsflüssigkeit (166) zu speichern; und
eine Pumpe (136; 186; 170; 203), die dazu ausgebildet ist, den Druck innerhalb des Behälters (108) zu modulieren, um einen positiven Gasdruck in dem Behälter bereitzustellen, um den Urin durch den Auslass (124) zu entlassen.

2. Flüssigkeitsübertragungsanordnung nach Anspruch 1, die weiter eine Auslassleitung (124) umfasst, die mit dem Auslass der Urinsammelvorrichtung gekoppelt ist und dazu ausgebildet ist, mit einem Abfallbehälter (162) in Fluidverbindung zu stehen.

3. Flüssigkeitsübertragungsanordnung nach einem der vorstehenden Ansprüche, wobei die Reinigungsflüssigkeit mindestens eines von Wasser oder einem Desinfektionsmittel einschließt.

4. Flüssigkeitsübertragungsanordnung nach einem der vorstehenden Ansprüche, wobei die Reinigungsvorrichtung einen ersten Motor (170), der dazu ausgebildet ist, positiven Luftdruck auf den Behälter der Urinsammelvorrichtung auszuüben, und einen zweiten Motor (186) einschließt, der dazu ausgebildet ist, die Flüssigkeit aus der Kammer in den Behälter zu übertragen.

5. Flüssigkeitsübertragungsanordnung nach einem der vorstehenden Ansprüche, wobei die Reinigungsvorrichtung eine Batterie einschließt, die dazu ausgebildet ist, die Reinigungsvorrichtung mit elektrischer Energie zu versorgen.

6. Flüssigkeitsübertragungsanordnung nach einem der vorstehenden Ansprüche, wobei die Reinigungsvorrichtung dazu ausgebildet ist, über die Urinsammelvorrichtung mit elektrischer Energie versorgt zu werden.

7. Flüssigkeitsübertragungsanordnung nach einem der vorstehenden Ansprüche, wobei die Urinsammelvorrichtung eine zweite Pumpe einschließt, die dazu ausgebildet ist, mit der Reinigungsvorrichtung in Fluidverbindung zu stehen.

8. Flüssigkeitsübertragungsanordnung nach einem der vorstehenden Ansprüche, wobei die Urinsammelvorrichtung einen Anschluss einschließt, der mit einem Vakuumschlauch und auch mit einer Leitung der Reinigungsvorrichtung koppelbar ist, um die Reinigungsflüssigkeit von der Reinigungsvorrichtung zu empfangen.

9. Flüssigkeitsübertragungsanordnung nach einem der vorstehenden Ansprüche, wobei die Pumpe dazu ausgebildet ist, positiven Druck auf den Behälter auszuüben, um die Reinigungsflüssigkeit aus dem Behälter zu entlassen, wirksam, um den Behälter von der Reinigungsflüssigkeit zu entleeren.

10. Flüssigkeitsübertragungsanordnung nach einem der vorstehenden Ansprüche, wobei die Urinsammelvorrichtung und die Reinigungsvorrichtung in einem gemeinsamen Gehäuse angeordnet sind.

11. Flüssigkeitsübertragungsanordnung nach Anspruch 1, wobei die Reinigungsvorrichtung weiter eine Luftkammer umfasst, die ein Luftvolumen einschließt.

12. Flüssigkeitsübertragungsanordnung nach Anspruch 11, wobei die Luftkammer mit der Umgebungsluft in Fluidverbindung steht.

13. Flüssigkeitsübertragungsanordnung nach einem der Ansprüche 11 oder 12, die weiter mindestens ein Ventil (180; 188) umfasst, das dazu ausgebildet ist, den Durchfluss der Flüssigkeit oder Luft durch einen Auslass der Reinigungsvorrichtung (130) zu regeln.

14. Flüssigkeitsübertragungsanordnung nach einem der Ansprüche 11 bis 13, wobei die Kammer, die Luftkammer, und die Pumpe innerhalb eines Gehäuses angeordnet sind.

15. Flüssigkeitsübertragungsanordnung nach einem der Ansprüche 11-14, wobei die Pumpe in einem Behälter der Reinigungsvorrichtung positioniert ist, wobei der Behälter dazu ausgebildet ist, Flüssigkeit zu speichern.

## Revendications

1. Ensemble de transfert de fluide (100 ; 200) pour la collecte et l'élimination d'urine, l'ensemble de transfert de fluide comprenant :
un dispositif (104) de collecte d'urine incluant :
un récipient (108) configuré pour stocker de l'urine collectée à partir d'un appareil (125) de collecte d'urine positionné à proximité d'une ouverture urétrale d'un individu, le récipient définissant un orifice pouvant être couplé à l'appareil de collecte d'urine ; et
une sortie configurée pour expulser l'urine du récipient ;
**caractérisé par**
un dispositif de nettoyage (130) en communication fluidique avec le récipient (108), le dispositif de nettoyage (130) incluant :
une chambre (134 ; 169 ; 205) configurée pour stocker un liquide de nettoyage (166) ; et
une pompe (136 ; 186 ; 170 ; 203) configurée pour moduler la pression à l'intérieur du récipient (108) pour fournir une pression de gaz positive dans le récipient pour expulser l'urine par la sortie (124).

2. Ensemble de transfert de fluide selon la revendication 1, comprenant en outre un conduit de sortie (124) couplé à la sortie du dispositif de collecte d'urine et configuré pour être en communication fluidique avec un récipient à déchets (162).

3. Ensemble de transfert de fluide selon l'une quelconque des revendications précédentes, dans lequel le liquide de nettoyage inclut au moins l'un parmi l'eau ou un désinfectant.

4. Ensemble de transfert de fluide selon l'une quelconque des revendications précédentes, dans lequel le dispositif de nettoyage inclut un premier moteur (170) configuré pour fournir une pression d'air positive au récipient du dispositif de collecte d'urine, et un deuxième moteur (186) configuré pour transférer le liquide de la chambre au récipient.

5. Ensemble de transfert de fluide selon l'une quelconque des revendications précédentes, dans lequel le dispositif de nettoyage inclut une batterie configurée pour alimenter le dispositif de nettoyage.

6. Ensemble de transfert de fluide selon l'une quelconque des revendications précédentes, dans lequel le dispositif de nettoyage est configuré pour recevoir de l'énergie via le dispositif de collecte d'urine.

7. Ensemble de transfert de fluide selon l'une quelconque des revendications précédentes, dans lequel le dispositif de collecte d'urine inclut une deuxième pompe configurée pour être en communication fluidique avec le dispositif de nettoyage.

8. Ensemble de transfert de fluide selon l'une quelconque des revendications précédentes, dans lequel le dispositif de collecte d'urine inclut un orifice pouvant être couplé à un tube à vide et également à un conduit du dispositif de nettoyage pour recevoir le liquide de nettoyage du dispositif de nettoyage.

9. Ensemble de transfert de fluide selon l'une quelconque des revendications précédentes, dans lequel la pompe est configurée pour fournir une pression positive au récipient pour expulser le liquide de nettoyage du récipient efficace pour vider le récipient du liquide de nettoyage.

10. Ensemble de transfert de fluide selon l'une quelconque des revendications précédentes, dans lequel le dispositif de collecte d'urine et le dispositif de nettoyage sont positionnés dans un boîtier commun.

11. Ensemble de transfert de fluide selon la revendication 1, dans lequel le dispositif de nettoyage comprend en outre une chambre à air incluant un volume d'air.

12. Ensemble de transfert de fluide selon la revendication 11, dans lequel la chambre à air est en communication fluidique avec l'atmosphère.

13. Ensemble de transfert de fluide selon l'une quelconque des revendications 11 ou 12, comprenant en outre au moins une vanne (180 ; 188) configurée pour réguler l'écoulement du liquide ou de l'air par une sortie du dispositif de nettoyage (130).

14. Ensemble de transfert de fluide selon l'une quelconque des revendications 11 à 13, dans lequel la chambre, la chambre à air et la pompe sont disposées à l'intérieur d'un boîtier.

15. Ensemble de transfert de fluide selon l'une quelconque des revendications 11 à 14, dans lequel la pompe est positionnée dans un réservoir du dispositif de nettoyage, le réservoir étant configuré pour stocker du liquide.
